# NEUE EUROPÄISCHE PATENTSCHRIFT

(11) **EP 0 644 777 B2**
(45) Veröffentlichungstag und Bekanntmachung des Hinweises auf die Entscheidung über den Einspruch: **15.10.2003**
(45) Hinweis auf die Patenterteilung: 11.02.1998
(21) Anmeldenummer: 93912897.1
(22) Anmeldetag: 05.06.1993
(51) Int. Cl.: A61K 49/00

(54) **MIKROPARTIKEL, VERFAHREN ZU DEREN HERSTELLUNG, SOWIE DIE VERWENDUNG DIESER IN DER DIAGNOSTIK**
MICROPARTICLES, METHOD OF PRODUCING THEM AND THEIR USE FOR DIAGNOSTIC PURPOSES
MICROPARTICULES, LEUR PROCEDE DE FABRICATION ET LEUR UTILISATION POUR ETABLIR DES DIAGNOSTICS

(30) Priorität: 13.06.1992 DE 4219723
(43) Veröffentlichungstag der Anmeldung: 29.03.1995
(73) Patentinhaber: SCHERING AKTIENGESELLSCHAFT, 13342 Berlin (DE)
(72) Erfinder: STEIN, Michael, Dr., D-10713 Berlin (DE); WEITSCHIES, Werner, Dr., D-10961 Berlin (DE); FRITZSCH, Thomas, Dr., D-12169 Berlin (DE); HELDMANN, Dieter, Dr., D-10555 Berlin (DE); SIEGERT, Joachim, Dr., D-12157 Berlin (DE); UHLENDORF, Volkmar, Dr., D-14050 Berlin (DE); HAMACHER, Esther, D-13507 Berlin (DE); LÜDERS, Frank, Dr., D-13051 Berlin (DE)
(86) Internationale Anmeldenummer: EP9301425
(87) Internationale Veröffentlichungsnummer: WO93025242

(56) Entgegenhaltungen:
- EP-A- 0 324 938
- EP-A- 0 327 490
- EP-A- 0 458 745
- EP-A- 0 512 693
- EP-A- 0 535 387
- WO-A-92/17514
- DE-A- 4 129 187
- DE-A- 4 209 487
- DE-A- 4 209 488
- US-A- 4 265 251
- US-A- 5 425 366
- ZA-A- 890 873
- STN FILE SUPPLIER & FILE MEDLINE AN=91297395 (KARLSRUHE)
- Harris, J.R., et al., Micron 26(2), 1995, Seiten 103-111
- Kinsler te al. in: Fundamentals of Acoustics, 3rd. Ed., 1982, John Wiley & Sons, Seiten 228-231
- Deasy Patrick B., "Microencapsulation and related Drug Processes", Ed. Marcel Dekker, 1984, chapter 10,
- M. Bornschein et al., in "Die Pharmazie", 44(9), 1989, Seiten 585-593
- Kawashima Y., et al., J. Pharm. Sci., 81 (2), 1992, Seiten 135-140
- Edwards, David A., et al., J. Appl. Physiol. 85, 1998, Seiten 379-385
- J. Barnhart, et al., Investigative Radiology, 25, 1990, Seiten S162-S164
- Eingabe vom 28/10/1996 der Fa. Nycomed aus dem Einspruch gegen EP 0 458 745, enthaltend die Gutachten von Rogstad (ER1) und Duus et al. (ER2)
- Schneider M., et al., Investigative Radiology, 26, 1991 Seiten S 190-191
- Lange H., Kolloid-Zeitschrift und Zeitschrift für Polymere, 223(1), 1968, Seiten 24-68
- Untersuchung Dr. Priewe (Schering AG)
- Erklaerung durch Dr.E. Mathiowitz
- Curriculum vitae von Dr. Howard Bernstein
- Vergleich der Versuchsprotokolle fuer A17 und A1(Beispiel 3)
- Erklaerung von Dr.Charles Church, enthaltend, Curriculum vitae, Nyborg 1978, Ultrasound: Its Applications in Medicine and Biology, pp. 39-41, Kinsler et al., 1982, Fundamentals of Accoustics, p. 461
- Kirk-Othmer, 1965, 2nd Ed., vol. 13, pp 436-456
- Wheatley et al., 1990, Biomaterials, 11, pp. 713-717
- Methoden der organischen Chemie, Houben-Weyl, Erweiterungs- und Folgebaende zur 4.ten Auflage,Seite 239, Gleichung 38, Seite 244, Referenz zu H.Lange unter Fussnote 2
- Techniques of Chemistry, vol. 1, Chapter II, Norman Bauer, Seymour Z. Levin, pp. 102, 104
- Methoden der analytischen Chemie, Bd.2, Teil 3, Rudolf Bock, Seite 42
- Table 2. Replication of Schering's Table 4 with the correct value for Da(=2.7 um),
- Density of Example 4 of A1 calculated using the distribution from A15
- Graphics representing he considerations for ultrasound contrast agents made in A23;

## Beschreibung

Die Erfindung betrifft den in den Patentansprüchen gekennzeichneten Gegenstand, d.h. Verfahren zur Herstellung von Mikropartikeln für spezielle Kontrastmittel für die Ultraschalldiagnostik deren Hülle aus Polycyanacrylaten oder Polyestern von α-β- oder γ-Hydroxycarbonsäuren aufgebaut ist, sowie die mit den Verfahren erkältlichen Mikropartikel.

Die mit erfindungsgemäßen verfahren erkältlichen Mikropartikel werden nachfolgend des erfindungsgemäße Partikel bzw. erfindungsgemäße Mikropartikel bezeichnet.

Es ist bekannt, daß durch periphere Injektion von Lösungen, die feine Gasblasen enthalten, cardiale Echokontraste erzielt werden können (Roelandt J, Ultrasound Med. Biol. 8: 471-492, 1982). Diese Gasblasen werden in physiologisch verträglichen Lösungen z. B. durch Schütteln, andere Agitation oder durch Zusatz von Kohlendioxid erhalten. Sie sind jedoch hinsichtlich Anzahl und Größe nicht standardisiert und können nur unzulänglich reproduziert werden. Auch sind sie in der Regel nicht stabilisiert, so daß ihre Lebensdauer gering ist Ihre mittleren Durchmesser liegen meist über Erythrocytengröße, so daß keine Lungenkapillarpassage mit nachfolgender Kontrastierung von Organen wie linkes Herz, Leber, Niere oder Milz möglich ist. Darüber hinaus eignen sie sich nicht für Quantifizierungen, da sich das von ihnen erzeugte Ultraschallecho aus mehreren, nicht voneinander zu trennenden Prozessen wie Blasenentstehung, Koaleszenz und Auflösung zusammensetzt. So ist es z. B. nicht möglich, mit Hilfe dieser Ultraschall-Kontrastmittel über die Messung des Kontrastverlaufs im Myokard Aussagen über die Transitzeiten zu gewinnen. Hierzu sind Kontrastmittel notwendig, deren Streukörper eine ausreichende Stabilität aufweisen.
In der EP 0 131 540 ist die Stabilisierung der Gasblasen durch Zucker beschrieben. Damit wird zwar die Reproduzierbarkeit und Homogenität des Kontrasteffektes verbessert, eine Lungenpassage überstehen diese Blasen jedoch nicht. In den EP 0 122 624 und 0 123 235 wird beschrieben, daß der gasblasenstabilisierende Effekt von Zuckern, Zuckeralkoholen und Salzen durch Zusatz von grenzflächenaktiven Substanzen verbessert wird. Eine Lungenkapillargängigkeit und die Möglichkeit zur Darstellung des arteriellen Gefäßschenkels und verschiedener Organe wie Leber oder Milz ist bei diesen Ultraschallkontrastmitteln gegeben. Der Kontrasteffekt ist hierbei jedoch auf das Gefäßlumen beschränkt, da die Bläschen nicht von den Gewebezellen aufgenommen werden. Keines der beschriebenen Ultraschall-Kontrastmittel verbleibt längere Zeit unverändert im Körper. Eine Organdarstellung mit ausreichender Signalintensität durch selektive Anreicherung nach i.v. Gabe oder Quantifizierungen sind mit diesen Mitteln nicht möglich.
Eine Verkapselung von Gasen, wie beispielsweise Luft als Ultraschall-Kontrastmittel wird in der EP 0 224 934 beschrieben. Das hierbei verwendete Wandmaterial besteht aus Protein, insbesondere menschliches Serumalbumin mit den bekannten allergenen Eigenschaften, zu denen durch eine Denaturierung cytotoxische Effekte hinzukommen können.

In der veröffentlichten Patentschrift EP 0 327 490 werden gasenthaltende Mikropartikel für die Uttraschatl-Diagnostik auf der Basis von biologisch abbaubaren, synthetischen Materialien beschrieben. Diese Mittel weisen eine ausreichende in-vivo Lebensdauer auf und werden nach intravenöser Applikation intrazellulär im Retikuloendothelialem System und damit auch in der Leber oder Milz angereichert. In dieser Schrift sind zwar unter anderem auch Polycyanacrylate bzw. α-, β- oder γ-Hydroxycarbonsäuren als geeignete Hüllmaterialien beansprucht, jedoch unterscheiden sich die erfindungsmäßen Partikel von den in der EP 0 327 490 offenbarten dadurch, daß die spezifische Dichte der Partikel < 0,7 g/cm³ ist.
Die geringere Dichte der erfindungsgemäßen Partikel resultiert daher, daß sie bei gleicher Größe mehr Gas enthalten. Da die Intensität des durch Streuung und Reflexion an einer Gasblase erzeugten Ultraschall echos von der sechsten Potenz des Gaskern-Radius abhängt, stellen die erfindungsgemäßen Partikel somit erheblich effektivere Uftraschall-Kontrastmittel als die in der EP 0 327 490 offenbarten Mittel dar.

Diese Vergrößerung des Gasvolumens wird erfindungsgemäß durch eine Verringerung der Wandstärke erreicht. Überraschenderweise sind die erfindungsgemäßen Partikel trotz der geringeren Wandstärke noch ausreichend stabil um auch die Lugenpassage zu überdauern.

Die neuen "dünnwandigen" Partikel lassen sich unter Verwendung der erfindungsgemäßen Verfahren herstellen. Die im Hinblick auf die Kapillargängigkeit geforderte Partiketgröße beträgt < 10 µm. Bevorzugt sind Partikel mit einem mittleren Durchmesser von 0,2 bis 2 µm. Diese Forderung wird bei Verwendung des erfindungsgemäßen Verfahrens ebenfalls erfüllt

Die spezifische Dichte der nach dem erfindungsgemäßen Verfahren hergestellten Partikel beträgt 0,05 g/cm³ bis 0,7 g/cm³, daraus errechnet sich eine mittlere Wandstärke der Partikel von 10 bis 50 nm.

Ein Vergleich der beispielhaft ausgewählten Polycyanacrylat-Partikel hergestellt nach Beispiel 9 der vorliegenden Erfindung, mit den nach Beispiel 2 der EP 0 327 490 hergestellten Partikeln, ergibt die in der *Tabelle 1* zusammengestellten Werte.

**Tabelle 1**

| | Partikel gemäß EP 0 327 490; hergestellt nach Beispiel 2 | erfindungsgemäße Partikel; hergestellt nach Beispiel 9 |
|---|---|---|
| Größe ca. | 1 µm | 0,8 µm |
| spez. Gewicht | 0,81 g/cm³ | 0,15 g/cm³ |
| mittlerer Durchmesser des Luftkerns^{*} | 0,64 µm | 0,76 µm |
| durchschnittliche mittlere Wandstärke | 180 nm | 20 nm |

| | | |
|---|---|---|
| *errechnet mit δ_{Polymer} =1,1 g/cm³ und δ_{Luft} ∼ 0 g/cm³ | | |

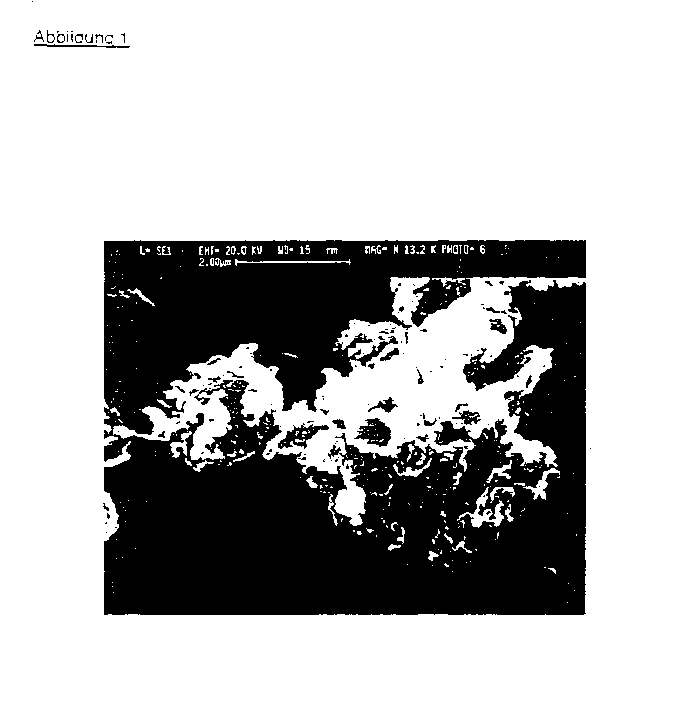

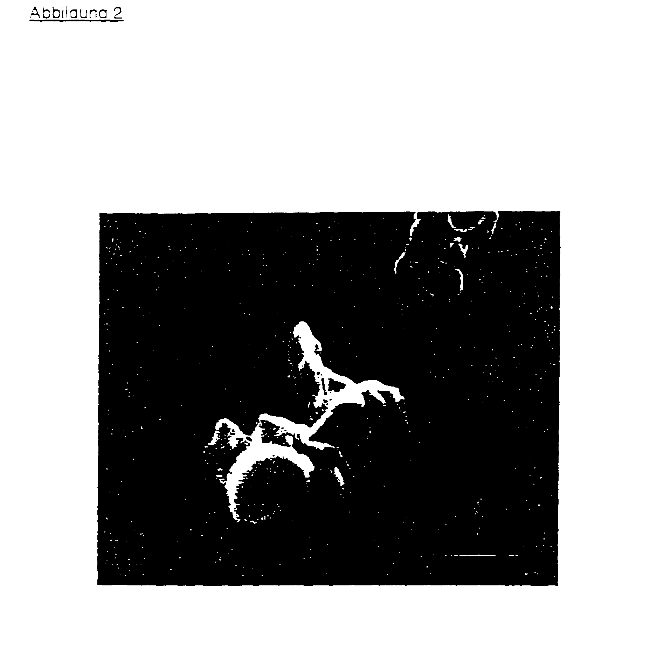

Aus dem spezifischen Gewicht der erfindungsgemäßen, nach Beispiel 9 hergestellten, Partikel von 0,15 g/cm³ errechnet sich eine um nahezu den Faktor 10 geringere mittlere Wandstärke und ein um ungefähr 60% größeres Gasvolumen.
Diese Werte sind in guter Übereinstimmung mit den Ergebnissen aus rasterelektronenmikroskopischen Aufnahmen [siehe Abbildung 1 (erfindungsgemäße Mikropartikel hergestellt nach Beispiel 9) und Abbildung 2 (Mikropartikel hergestellt gemäß EP 0327 490 / Beispiel 2)].

Überraschenderweise wird im Falle der erfindungsgemäßen Partikel bei Ultraschalluntersuchungen über die streuungsbedingte Signalverstärkung hinaus, eine weitere Kontraststeigerung durch die Partikel beobachtet, da sie durch Einstrahlung von Ultraschall geeigneten Schalldrucks und geeigneter Frequenz zu eigenständigen Signalen angeregt werden.
Aufgrund dieses unerwartet beobachteten Phänomens ergibt sich erstmals ein neues Anwendungsgebiet für die erfindungsgemäßen Mittel bei der Tumordiagnose im Leber- und Milzbereich. So erscheint mit farbcodierter Dopplertechnik nach peripherer venöser Gabe der erfindungsgemäßen Mikropartikel, gesundes Gewebe farbig, während Tumorbezirke weniger oder nicht farbcodiert erscheinen.

Weiterhin lassen sich aufgrund der beschriebenen vorteilhaften Partikeleigenschaften - größeres Gasvolumen plus eigenständige Ultraschallsignale - bereits mit einer erheblich geringeren Partikelzahl gute Kontraste erzielen. Als Folge dieser gesteigerten Effektivität sind auch nur geringe Mengen (im µg-Bereich) an polymerer Substanz für einen guten Ultraschallkontrast nötig, wodurch sich der pharmakologische Sicherheitsabstand erhöht.

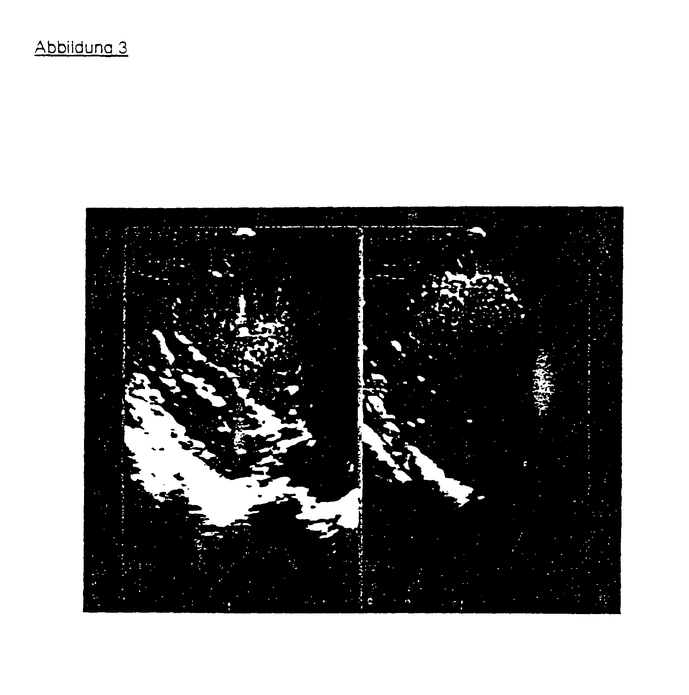

So ergeben in einem typischen Tierversuch am Beagle bereits 25 µg der erfindungsgemäßen Partikel hergestellt nach Beispiel 9 pro kg Körpergewicht (KGW) einen optimalen, homogenen Kontrast der linken Herzhöhlen (*Abbildung 3* / *rechts*). Mit den Partikeln der EP 0 327 490 (hergestellt nach Beispiel 2) war erst bei einer Dosierung von 2 mg/kg KGW ein vergleichbarer Kontrast zu erzielen (*Abbildung 3* / *links*).

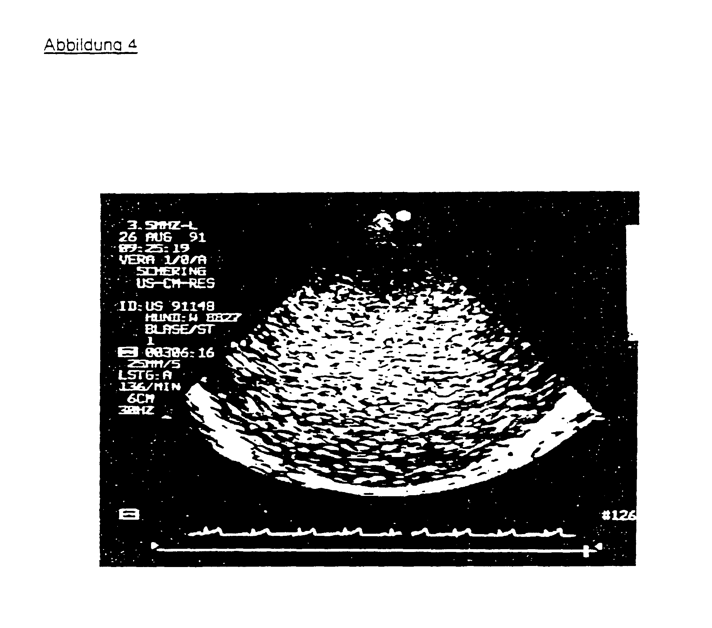

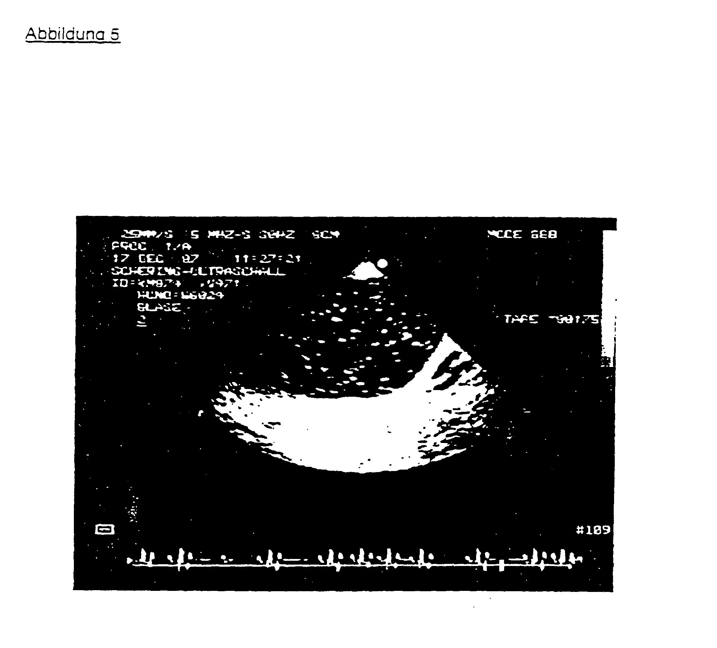

ln *Abbildung 4* sind die Kontrasteffekte der hier offenbarten Partikel (Beispiel 9), in Abbildung 5 die der in EP 0 327 490 beschriebenen Partikel (Beispiel 2) nach Instillation in die Base dargestellt. Die Partikel der EP 0 327 490 wurden in einer Dosierung von ca. 3 mg/ml, die erfindungsgemäßen Partikel mit 0,04 mg/ml gegeben. Man erkennt, daß trotz deutlich geringerer Konzentration ein erheblich besserer Kontrasteffekt erreicht wird.

Ein weiterer Aspekt der Erfindung betrifft Verfahren zur Herstellung der erfindungsgemäßen Mikropartikel. Zur Herstellung von Mikropartikeln auf der Basis von Polycyanacrylat verfährt man in der Weise, daß monomeres Cyanacrylat in einer sauren, mit einem Gas gesättigten, wässrigen Lösung, die gegebenenfalls mindestens eine oberflächenaktive Substanz enthält, mit einem Rotor-Stator-Mischer dispergiert wird, die nach 5 Minuten bis 3 Stunden Dispergierung erhaltenen Partikel abgetrennt werden, gegebenenfalls mit Wasser gewaschen werden, anschließend in einem pharmazeutisch akzeptablen Suspensionsmedium aufgenommen und gefriergetrocknet werden, wobei die Suspension vorteilhafterweise während des Einfrierens kräftig bewegt wird. Vorzugsweise wird als Cyanacrylat der Butylester, als Gas Luft, Stickstoff, Sauerstoff, Edelgase oder Kohlendioxid eingesetzt. Anstelle des Rotor-Stator-Mischers können auch vergleichbare Geräte (wie z.B. ein Dissolver-Rührer) verwendet werden, die ein kräftiges Dispergieren der Mischung erlauben. Als oberflächenaktive Substanz wird (werden) bevorzugt (eine) Substanz(en) aus der Gruppe Polysorbate, Octyl- oder Nonylphenole, Macrogol-glycerolester oder Cetomacrogole oder Poloxamere ® oder deren Gemische, verwendet. Der pH-Wert der wässrigen gasgesättigten Lösung liegt vorzugsweise zwischen 1,8 und 4,5, zur Einstellung des pH-Wertes eignen sich insbesondere Salz- und Phosphorsäure. Die Abtrennung der Partikel erfolgt mittels Zentrifugation oder Flotation. Als Suspensionsmedium eignet sich Wasser für Injektionszwecke gegebenenfalls mit einem Zusatz von Kochsalz und/oder Glucose und/oder Mannitol und/oder Lactose, das gegebenenfalls zusätzlich noch eine oberflächenaktive Substanz, wie z.B. aus der Gruppe der Polysorbate, Octyl- oder Nonylphenole, Macrogolglycerolester oder Cetomacrogole oder Substanzen aus der Gruppe der Poloxamere ® oder deren Gemische und/oder einen mehrwertigen Alkohol enthält.

Zur Herstellung von Mikropartikeln auf der Basis von Polyestern verfährt man in der Weise, daß ein Polyester einer α-,β- oder γ-Hydroxycarbonsäure, gegebenenfalls gemeinsam mit einem wasser-dispergierbaren Emulgator, in einem gesundheitlich unbedenklichen Lösungsmittel gelöst wird und diese Lösung unter Dispergierung mit einem Dissolver Rührer oder einem Schallstab zu einer gashaltigen Flüssigkeit gegeben wird, die sofern der Emulgator nicht bereits gemeinsam mit dem Polyester zugesetzt wurde, einen wasser-dispergierbaren Emulgator enthält, die nach 30 Minuten bis 2 Stunden Dispergierung erhaltenen Partikel abgetrennt werden, gegebenenfalls mit Wasser gewaschen werden, anschließend in einem pharmazeutisch akzeptablen Suspensionsmedium aufgenommen und gefriergetrocknet werden.

Erfindungsgemäß bevorzugt sind Polymere der Milchsäure oder der Glycolsäure sowie deren Mischpolymerisate. Als unbedenkliches Lösungsmittel wird vorzugsweise erhitzter Ethylalkohol verwendet. Als gashaltige Flüssigkeit wird vorzugsweise Wasser oder Glycerol 87 % verwendet, die bevorzugten Gase sind Luft, Stickstoff, Sauerstoff, Edelgase oder Kohlendioxid. Als wasser dispergierbarer Emulgator sind Phosphatidycholin oder Sucrose-Palmitat-Stearat 15 sowie deren Gemische zu nennen. Als pharmazeutisch akzeptables Suspensionsmedium eignen sich dieselben Medien wie im Falle der Partikel auf der Basis von Polycyanacrylat.

Die Gefriertrocknung der erfindungsgemäßen Mikropartikel erfolgt vorteilhafterweise unter Zusatz von Substanzen, die die Partikel bei der Gefriertrocknung vor Zerstörung und/oder Agglomeration schützen (sog. Kryoprotektoren). Als Kryoprotektoren können der Suspension vorteilhafterweise Biopolymere (z. B. Albumin, autoklavierte Gelatine, Oxypolygelatine, Gelatine-polysuccinat, vernetzte Polypeptide), synthetische makromolekulare Substanzen (beispielsweise Povidone, Polyvinylalkohol), Zucker (z.B. Saccharose, Lactose, Trehalose, Raffinose), Zuckeralkohole (z.B. Mannitol, Sorbitol) oder Mischungen dieser pharmazeutischen Hilfsstoffe in einer Konzentration von 1 % bis 15 % zugesetzt werden. Der erfindungsgemäße Zusatz der Kryoprotektoren erfolgt entweder zum Herstellungsmedium, durch Aufnahme der Mikropartikel nach der Abtrennung durch Flotation in der Kryoprotektorlösung oder durch Zugabe zur Suspension unmittelbar vor der Gefriertrocknung.

Überraschenderweise wurde gefunden, daß es vorteilhaft sein kann, zusätzlich zum Kryoprotektor eine die Gefriertrocknung optimierende Substanz aus der Gruppe der Polyole (z.B. Glycerol, Propylenglycol) oder DMSO in einer Konzentration von 0,1 bis 3 % zuzusetzen.

Die Gefriertrocknung erfolgt vorteilhafterweise so, daß eine Flotation der erfindungsgemäßen Mikropartikel beim Einfrieren verhindert wird. Dazu ist es vorteilhaft, die Suspension der erfindungsgemäßen Mikropartikel vorzukühlen, mit einer Einfriergeschwindigkeit von 2 Kelvin je Minute oder mehr einzufrieren und gefrierzutrocknen.

Ein weiterer wesentlicher Vorteil der erfindungsgemäßen Herstellungsverfahren gegenüber den in der EP 0 327 490 offenbarten Methoden ist, daß sich die gasgefüllten Partikel ohne Verwendung eines gesundheitlich und ökologisch bedenklichen organischen Lösungsmittels oder Hilfsstoffes in einem einzigen Verfahrensschritt herstellen lassen.

Die Herstellung der gebrauchsfertigen, injizierbaren Zubereitung der erfindungsgemäßen Partikel, erfolgt durch Resuspendieren des Lyophilisats in einem pharmazeutisch akzeptablen Suspensionsmedium wie z.B. Wasser p.i., wäßrige Lösungen eines oder mehrerer anorganischer Salze wie physiologische Elektrolyt-Lösungen und Pufferlösungen wie z.B Tyrode, wäßrige Lösungen von Mono- oder Disacchariden wie Glucose oder Lactose, Zuckeralkoholen wie Mannit, die gegebenenfalls zusätzlich noch eine oberflächenaktive Substanz z.B. aus der Gruppe der Polysorbate, Octyl- oder Nonylphenole, Macrogolglycerolester oder Cetromacrogole oder Substanzen aus der Gruppe der Poloxamere oder deren Gemischen und/oder einem physiologisch verträglichen mehrwertigen Alkohol wie Glycerin, enthalten, bevorzugt jedoch in für Injektionszwecke geeignetem Wasser. Die Gesamtkonzentration der gegebenenfalls gelösten Stoffe beträgt 0-15 Gewichts-Prozent.

Ein alternatives Verfahren zur Herstellung der gebrauchsfertigen, injizierbaren Zubereitungen besteht darin, daß bei dem erfindungsgemäßen Verfahren - zur Herstellung der Mikropartikel - auf die abschließende Gefriertrocknung verzichtet wird. Um die Sicherheit der Applikation zu erhöhen, kann unmittelbar vor Injektion eine Filtration der Suspension durchgeführt werden. Dies geschieht vorteilhafterweise durch ein zwischen Spritze und Kanüle angebrachtes Filter, das dazu dient, bei Handhabungsfehlern eventuell auftretende Aggregate zurückzuhalten, nichtaggregierte Partikel jedoch passieren zu lassen. Als Filtermaterial eignen sich prinzipiell die handelsüblichen Membranfilter. Bei der Auswahl der Filter zeigte sich jedoch überraschenderweise, daß die besten Resultate mit speziellen mehrlagigen Membranen aus Polypropylen-Mikrofäden erzielt werden. Besonders vorteilhaft erwiesen sich Filter mit einer absoluten Rückhafterate von 10-20 µm. Diese Filter sind in der Lage, auch kleinere, z.B. zwischen 5 und 10 µm große, bei einer intravenösen Anwendung jedoch unerwünschte Partikelaggregate, zurückzuhalten.

Die Konzentration des gebrauchsfertigen Kontrastmittels kann im Bereich von 0,1 bis 20 mg, bevorzugt von 2 bis 6 mg Partikel/ml Suspensionsmedium eingestellt werden. Die injizierte Dosis ist abhängig von der gewünschten Anwendung und liegt z.B. in der Farbdoppler-Sonographie bei der Untersuchung der Gefäße im Bereich 1 bis 500, bevorzugt zwischen 10 und 100 µg Partikel/kg Körpergewicht, bei der Untersuchung von Leber und Milz im Bereich von 50 bis 1000, bevorzugt zwischen 200 und 600 µg/kg Körpergewicht.

Die Erfindung wird durch folgende Beispiele erläutert:

### Beispiel 1:

0,4 ml Cyanacrylsäurebutylester werden in 60 ml HCl von pH 2,0, die 1 % Poloxamer 407 enthält, mit einem Rotor - Stator - Mischer 5 min lang dispergiert. Die Mikropartikel mit einer durchschnittlichen Größe von 2 µm werden abzentrifugiert und in 300 ml einer wässrigen Lösung von 1 % Poloxamer und 5 % Glucose aufgenommen. Die Dichtebestimmung ergibt ein spezifisches Gewicht von 0,2 g / cm³.

### Beispiel 2:

Es wird wie in Beispiel 1 verfahren, wobei die Salzsäure einen pH-Wert von 2,5 aufweist und Poloxamer 407 durch Octoxynol - 9 ersetzt wird. Die Mikropartikel besitzen eine durchschnittliche Größe von etwa 0,9 µm und ein spezifisches Gewicht von 0,2 g / cm³. Sie werden in 300 ml 5 %iger Mannitol - Lösung, die 0,1 % Polysorbat 20 enthält, aufgenommen.

### Beispiel 3:

Es wird wie in Beispiel 1 verfahren, wobei die Salzsäure einen pH-Wert von 3,0 aufweist und Poloxamer 407 durch Cetomacrogol 1200 ersetzt wird. Die Durchschnittsgröße der Mikropartikel beträgt 1,5 µm, ihr spezifisches Gewicht 0,3 g / cm³. Sie werden in 300 ml 5 %iger Mannitol - Lösung, die 0,1 % Cetomacrogol 1200 und 5 % Povidone enthält, aufgenommen.

### Beispiel 4:

Es wird wie in Beispiel 1 verfahren, wobei Poloxamer 407 durch 5 % Polysorbat 40 ersetzt wird. Die Durchschnittsgröße der Mikropartikel beträgt 1,0 µm, ihr spezifisches Gewicht 0,4 g/cm³. Sie werden in 300 ml 5 %iger Mannitol - Lösung, die 1 % Macrogolglycerolhydroxystearat enthält, aufgenommen.

### Beispiel 5:

Es wird wie in Beispiel 1 verfahren, wobei Poloxamer 407 durch 5 % Macrogolglycerolhydroxystearat ersetzt wird. Die Partikel sind durschnittlich 0.9 µm groß und haben ein spezifisches Gewicht von 0,3 g/cm³. Sie werden in 300 ml 5 %iger Mannitol - Lösung, die 1 % Macrogolglycerolhydroxystearat und 10 % Propylenglykol enthält, aufgenommen.

### Beispiel 6:

Es wird wie in Beispiel 1 verfahren, wobei Cyanacrylsäurebutylester durch Cyanacrylsäureethylester ersetzt wird. Die Mikropartikel haben eine durchschnittliche Größe von 1,5 µm, und ein spezifisches Gewicht 0,2 g/cm³. Sie werden in 300 ml einer wässrigen Lösung von 1 % Poloxamer 407 und 5 % Glucose aufgenommen.

### Beispiel 7:

Es wird wie in Beispiel 1 verfahren, wobei Cyanacrylsäurebutylester durch Cyanacrylsäureisopropylester ersetzt wird. Die Mikropartikel haben eine durchschnittliche Größe von 1,3 µm, und ein spezifisches Gewicht 0,2 g/cm³. Sie werden in 300 ml einer wässrigen Lösung von 1 % Poloxamer 407 und 5 % Mannitol und 10 % Propylenglykol aufgenommen.

### Beispiel 8:

3 ml Cyanocrylsäurebutylester werden in 300 ml HCl von pH 2,0, die 1 % Polaxamer 407 enthält, mit einem Dissolver - Mischer 120 min lang dispergiert. Die Mikropartikel mit einer durchschnittlichen Größe von 2 µm und einem spezifischen Gewicht von 0,1 g/cm³ werden durch Flotation abgetrennt und in 51 einer 5 %igen Mannitol - Lösung, die 1 % Poloxamer 407 und 10 % Propylenglykol enthält, aufgenommen.

### Beispiel 9:

Es wird wie in Beispiel 8 verfahren, wobei Poloxamer 407 durch Octoxynol - 9 ersetzt und der pH-Wert auf 2,5 eingestellt wird. Die Durchschnittsgröße der Mikropartikel beträgt 0,8 um, ihr spezifisches Gewicht 0,15 g/cm³. Sie werden in 5l einer 0,9 %igen Kochsatzlösung, die 0,1 % Cetomacrogol 1200 enthält, aufgenommen.

### Beispiel 10:

Es wird wie in Beispiel 8 verfahren, wobei Poloxamer 407 durch Cetomacrogol 1200 ersetzt wird. Die Durchschnittsgröße der Mikropartikel beträgt 1,8 µm, ihr spezifisches Gewicht 0,4 g/cm³. Sie werden in 5l einer 5 %igen Glucoselösung, die 0,2 % Cetomacrogol 1200 enthält, aufgenommen.

### Beispiel 11:

Es wird wie in Beispiel 8 verfahren, wobei Poloxamer 407 durch 5 % Polysorbat 60 ersetzt wird. Die Durchschnittsgröße der Mikropartikel beträgt 1,0 µm, ihr spezifisches Gewicht 0,4 g/cm³. Sie werden in 5 l einer 5 %igen Mannitolllösung, die 1 % Poloxamer 407 und 10 % Propylenglykol enthält, aufgenommen.

### Beispiel 12:

Die in Beispiel 8, 9, 10 oder 11 hergestellten Partikel werden statt in dort angegebenen Lösungen in je 5l einer 5 %igen Mannitol - Lösung, die 0,1 % Cetomacrogol 1200 und 5 % Povidone enthält, aufgenommen, in 15 ml - Portionen unter kräftigem Schütteln eingefroren und gefriergetrocknet. Vor Anwendung wird das Lyophilisat mit Wasser für Injektionszwecke resuspendiert und gegebenfalls filtriert.

### Beispiel 12 a:

Die in Beispiel 8, 9, 10 oder 11 hergestellten Partikel werden statt in den dort angegebenen Lösungen in 5l einer 10 %igen Lactose-Lösung. die 0,1 % Cetomacrogol 1200 enthält, aufgenommen, in 15 ml-Portionen unter kräftigem Schütteln eingefroren und gefriergetrocknet. Vor der Anwendung wird das Lyophilisat mit Wasser für lnjektionswecke resuspendiert und gegebenenfalls filtriert.

### Beispiel 13:

1,0 g hydriertes Sojalecithin werden in 200 ml Glycerol mit einem Dissolver-Mischer dispergiert. Zu der Dispersion werden nach 60 min 2,0 g in 10 ml siedendem Ethanol gelöstes Poly-L-Lactid (mittleres Molekulargewicht 1100) zugetropft. Es wird 60 min lang weiterdispergiert. Die entstandenen Mikropartikel werden bei 1000 Upm zentrifugiert, der Überstand in 50 ml Wasser aufgenommen, erneut zentrifugiert (1000 Upm), der Überstand wird in 5 %iger Mannitol-Lösung aufgenommen. Diese Suspension wird in 10 ml Portionen aufgeteilt und gefriergetrocknet. Das Lyophilisat wird vor Anwendung mit Wasser für Injektionszwecke resuspendiert.

### Beispiel 14:

1,0 g Sucrose-Palmitat-Stearat (HLB 15) werden in 200 ml Glycerol mit einem Dissolver-Mischer dispergiert. ln die Dispersion wird nach 30 min 1,0 g in 10 ml siedendem Ethanol gelöstes Poly-L-Lactid (mittleres Molekulargewicht 1100) zugetropft. Es wird 60 min weiterdispergiert. Die entstandenen Mikropartikel haben eine durchschnittliche Größe von 2 µm. Sie werden bei 1000 Upm 30 min lang zentrifugiert, der Überstand wird in 50 ml Wasser aufgenommen, erneut zentrifugiert (1000 Upm), der Überstand wird in 50 ml einer 5 %igen Mannitol-Lösung aufgenommen. Diese Suspension wird in 10 ml-Portionen aufgeteilt und gefriergetrocknet. Das Lyophilisat wird vor Anwendung mit Wasser für Injektionszwecke resuspendiert.

### Beispiel 15:

Einem Hund (12,5 kg, Inhalationsnarkose) werden nach Beispiel 8 hergestellte Mikropartikel (250 µg/ml)in einer Dosis von 25 µg/kg Körpergewicht peripher-venös mit einer Geschwindigkeit von 2 ml/min injiziert. ln der linken Herzhälfte zeigt sich bei der Ultraschalluntersuchung im B-Bild eine kräftige, langanhattende Signalverstärkung.

### Beispiel 16:

Man wiederholt Beispiel 15 mit den in den Beispielen 1 - 7 oder 9 - 14 hergestellten Partikeln. Auch hierbei zeigen sich kräftige, langanhaltende Signalverstärkungen in der linken Herzhälfte.

### Beispiel 17:

Einem Hund (11 kg, Inhalationsnarkose) werden nach Beispiel 8 hergestellte Mikropartikel (250 µg/ml) in einer Dosis von 300 µg / kg Körpergewicht peripher - venös mit einer Geschwindigkeit von 0,1 ml/s injiziert. Nach 10 min stellt sich die Leber bei der Farbdoppleruntersuchung über einen zur Untersuchung ausreichend langen Zeitraum homogen farbcodiert dar.

### Beispiel 18:

Man wiederholt Beispiel 17 mit den in den Beispielen 1 - 7 oder 9 - 14 hergestellten Partikeln. Auch hierbei stellt sich die Leber homogen farbcodiert dar.

### Beispiel 19:

Die in Beispiel 8 hergestellten Mikropartikel werden 1 + 1 mit Hundeserum gemischt und bei 37 °C inkubiert. Noch 4 Stunden ist die vorher trübe Suspension völlig klar, und es finden sich mittels GC fast 100 % der theoretisch zu erwartenden Butanolmenge.

### Beispiel 20:

3 ml Cyanacrylsäurebutylester werden in 300 ml HCl von pH 2,5, die 1% Nonoxynol enthält, mit einem Dissolver-Mischer 90 min lang dispergiert. Die Mikropartikel mit einer durchschnittlichen Größe von 1,4 µm werden durch Flotation abgetrennt, in 100 ml Wasser aufgenommen und dann mit 5% Albumin versetzt. Anschließend wird unter leichter Bewegung in 5 ml-Portionen eingefroren und gefriergetrocknet.

### Beispiel 21:

3 ml Cyanacrylsäurebutylester werden in 300 ml HCl von pH 2,5, die 1% Octoxynol enthält, mit einem Dissolver-Mischer 90 min lang dispergiert. Die Mikropartikel mit einer durchschnittlichen Größe von 1,4 µm werden durch Flotation abgetrennt, in 100 ml mit HClNaOH auf pH 5,0 eingestellter 5%iger autoklavierter Gelatinelösung aufgenommen und anschließend unter leichter Bewegung in 5 ml-Portionen eingefroren sowie gefriergetrocknet.

### Beispiel 22:

3 ml Cyanacrylsäurebutylester werden in 300 ml HCl von pH 2,5, die 1% Octoxynol und 5% Povidone enthält, mit einem Dissolver-Mischer 90 min lang dispergiert. Die Mikropartikel mit einer durchschnittlichen Größe von 1,4 µm werden durch Flotation abgetrennt, in 5%iger Povidon-Lösung aufgenommen und in 5 ml-Portionen abgefüllt. Anschließend wird die abgefülfte Suspension eine Stunde auf 0°C temperiert und eingefroren sowie gefriergetrocknet.

### Beispiel 23:

3 ml Cyanacrylsäurebutylester werden in 300 ml HCl von pH 2,5, die 1% Octoxynol enthält, mit einem Dissolver-Mischer 90 min lang dispergiert. Die Mikropartikel mit einer durchschnittlichen Größe von 1,4 µm werden durch Flotation abgetrennt, in 300 ml Wasser aufgenommen, mit 0,1% Glycerol und 10% Lactose versetzt und nach Einfrieren gefriergetrocknet.

## Patentansprüche

1. Verfahren zur Herstellung von Mikropartikeln, **dadurch gekennzeichnet, daß** monomeres Cyanacrylat in einer sauren, mit einem Gas gesättigten, wässrigen Lösung, die gegebenenfalls mindestens eine oberflächenaktive Substanz enthält dispergiert wird, die nach 5 Minuten bis 3 Stunden Dispergierung erhaltenen Partikel abgetrennt werden, gegebenenfalls mit Wasser gewaschen werden, anschießend in einem pharmazeutisch akzeptablen Suspensionsmedium aufgenommen und gefriergetrocknet werden.

2. Verfahren zur Herstellung von Mikropartikeln, **dadurch gekennzeichnet, daß** ein Polyester einer α-, β- oder γ-Hydroxycarbonsäure, gegebenenfalls gemeinsam mit einem wasser-dispergierbaren Emulgator, in einem gesundheitlich unbedenklichen Lösungsmittel gelöst wird und diese Lösung unter Dispergierung zu einer gashaltigen Flüssigkeit gegeben wird, die sofern der Emulgator nicht bereits gemeinsam mit dem Polyester zugesetzt wurde, einen wasser-dispergierbaren Emulgator enthält die nach 30 Minuten bis 2 Stunden Dispergierung erhabenen Partikel abgetrennt werden, gegebenenfalls mit Wasser gewaschen werden, anschließend in einem pharmazeutisch akzeptablen Suspensionsmedium aufgenommen und gefriergetrocknet werden.

3. Mikropartikel erhältlich nach dem in Anspruch 1 genannten Verfahren.

4. Mikropartikel erhältlich nach dem in Anspruch 2 genannten Verfahren.

5. Verfahren zur Herstellung von Mikropartikeln gemäß Anspruch 1, **dadurch gekennzeichnet. daß** der pH-Wert der wässrigen Lösung zwischen 1,8 und 4,5 liegt.

6. Verfahren zur Herstellung von Mikropartikeln gemäß Anspruch 5, **dadurch gekennzeichnet, daß** zur Einstellung des pH-Wertes Salz-oder Phosphorsäure verwendet wird.

7. Verfahren zur Herstellung von Mikropartikeln gemäß Anspruch 1, **dadurch gekennzeichnet daß** als oberflächenaktive Substanzen Polysorbate, Octyl- oder Nonylphenole, Macrogol-glycerolester oder Cetomacrogole oder Substanzen aus der Gruppe der Polaxamere ® oder deren Gemische, verwendet werden.

8. Verfahren zur Herstellung von Mikropartikeln gemäß Anspruch 2, **dadurch gekennzeichnet daß** als wasser-dispergierbare(r) Emulgator(en) Phosphatidycholin oder Sucrose-Palmitat-Stearat 15 oder deren Gemische verwendet wird (werden)

9. Verfahren zur Herstellung von Mikropartikeln gemäß Anspruch 2, **dadurch gekennzeichnet, daß** als gesundheitlich unbedenkliches Lösungsmittel für den Polyester ein niederer Alkohol, bevorzugt Ethylalkohol verwendet wird.

10. Verfahren zur Herstellung von Mikropartikeln gemäß Anspruch 2, **dadurch gekennzeichnet. daß** als gashaltige Flüssigkeit Wasser oder Glycerol 87 % verwendet wird.

11. Verfahren zur Herstellung von Mikropartikeln gemäß Anspruch 1 bzw 2, **dadurch gekennzeichnet, daß** als Suspensionsmedium Wasser für Injektionszwecke gegebenenfalls mit einem Zusatz von Kochsalz und/oder Glucose und/oder Mannitol und/oder Lactose, das gegebenenfalls zusätzlich noch eine oberflächenaktive Substanz gemäß Anspruch 13 und/oder einen mehrwertigen Alkohol enthält, verwendet wird.

12. Ultraschallkontrastmittel, **dadurch gekennzeichnet, daß** die Mikropatikel nach Anspruch 3 bzw 7 in einem pharmazeutisch akzeptablen Suspensionsmedium resuspendiert werden.

13. Ultraschallkontrastmittel gemäß Anspruch 12, **dadurch gekennzeichnet, daß** als pharmazeutisch akzeptables Suspensionsmedium Wasser für Injektionszwecke, das gegebenenfalls einen Zusatz von Kochsalz, Glucose, Mannitol und/oder Lactose und gegebenenfalls zusätzlich noch eine oberflächenaktive Substanz gemäß Anspruch 13 und/oder mehrwertigen Alkohol enthält, verwendet wird.

14. Ultraschallkontrastmittel herstellbar nach dem in den Ansprüchen 1 und 2 beschriebenen Verfahren, **dadurch gekennzeichnet, daß** auf eine abschließende Gefriertrocknung verzichtet wird

## Claims

1. Process for the production of microparticles, **characterised in that** monomeric cyanoacrylate is dispersed in an acidic, gas-saturated, aqueous solution optionally containing at least one surface-active substance, the particles obtained after dispersion for from 5 minutes to 3 hours are isolated, optionally washed with water, then taken up in a pharmaceutically acceptable suspension medium and freeze-dried.

2. Process for the production of microparticles, **characterised in that** a polyester of an α-, β- or γ-hydroxycarboxylic acid, optionally together with a water-dispersible emulsifier, is dissolved in a physiologically harmless solvent, and that solution is added, with dispersion, to a gas-containing liquid that contains, it the emulsifier has not already been added together with the polyester, a water-dispersible emulsifier, the particles obtained alter dispersion for from 30 minutes to 2 hours are isolated, optionally washed with water, then taken up in a pharmaceutically acceptable suspension medium and freeze-dried.

3. Microparticles obtainable by the process mentioned in claim 1.

4. Microparticles obtainable by the process mentioned in claim 2.

5. Process for the production of microparticles according to claim 1, **characterised in that** the pH value of the aqueous solution is from 1.8 to 4.5.

6. Process for the production of microparticles according to claim 5, **characterised in that** hydrochloric acid or phosphoric acid is used to adjust the pH value.

7. Process for the production of microparticles according to claim 1, **characterised in that** polysorbates, octyl or nonyl phenols, macrogol glycerol esters or cetamacrogols or substances from the group of the Poloxamers® or mixtures thereof are used as the surface-active substances.

8. Process for the production of microparticles according to claim 2, **characterised in that** phosphatidylcholine or sucrose-patmitate-stearate 15 or mixtures thereof is(are) used as water-dispersible emulsifier(s).

9. Process for the production of microparticles according to claim 2, **characterised in that** a lower alcohol, preferably ethyl alcohol, is used as the physiologically harmless solvent for the polyester.

10. Process for the production of microparticles according to claim 2, **characterised in that** water or 87 % glycerol is used as the gas-containing liquid.

11. Process for the production of microparticles according to claim 1 or 2, **characterised in that** there is used as the suspension medium water for injection purposes optionally with the addition of sodium chloride and/or glucose and/or mannitol and/or lactose, which optionally contains, in addition, a surface-active substance according to claim 13 and/or a polyhydric alcohol.

12. Ultrasound contrast medium, **characterised in that** the microparticles according to claim 3 or 4 are resuspended in a pharmaceutically acceptable suspension medium.

13. Ultrasound contrast medium according to claim 12, **characterised in that** there is used as the pharmaceutically acceptable suspension medium water for injection purposes which optionally contains an addition of sodium chloride, glucose, mannitol and/or lactose and optionally, in addition, a surface-active substance according to claim 7 and/or a polyhydric alcohol.

14. Ultrasound contrast medium capable of being produced by the process described in claims 1 and 2, **characterised in that** final freeze-drying is dispensed with.

## Revendications

1. Procédé de préparation de microparticules, **caractérisé en ce que** du cyanoacrylate monomère est dispersé dans une solution aqueuse, acide, saturée par un gaz, laquelle solution contient éventuellement au moins une substance tensioactive, **en ce que** les particules obtenues après 5 minutes à 3 heures de dispersion sont séparées, sont éventuellement lavées avec de l'eau, reprises ensuite dans un milieu de suspension pharmaceutiquement acceptable et lyophilisées.

2. Procédé de préparation de microparticules, **caractérisé en ce qu'**un polyester d'un acide α-, β- ou γ-hydrocarboxylique, éventuellement ensemble avec un émulsifiant dispersible dans l'eau, est dissous dans un solvant inoffensif du point de vue de la santé et **en ce que** cette solution est ajoutée en dispersant à un liquide contenant du gaz qui contient un émulsifiant dispersible dans l'eau, dans la mesure où l'émulsifiant n'a pas déjà été ajouté avec le polyester, **en ce que** les particules obtenues après 30 min à 2 heures de dispersion sont séparées, sont éventuellement lavées avec de l'eau et sont ensuite reprises dans un milieu de suspension pharmaceutiquement acceptable et lyophilisées.

3. Microparticules que l'on peut obtenir selon le procédé décrit dans la revendication 1.

4. Microparticules que l'on peut obtenir selon le procédé décrit dans la revendication 2.

5. Procédé de préparation de microparticules selon la revendication 1, **caractérisé en ce que** la valeur du pH de la solution aqueuse est comprise entre 1,8 et 4,5.

6. Procédé de préparation de microparticules selon la revendication 5, **caractérisé en ce que**, pour régler la valeur du pH, on utilise de l'acide chlorhydrique ou de l'acide phosphorique.

7. Procédé de préparation de microparticules selon la revendication 1 , **caractérisé en ce qu'**on utilise, comme substances tensioactives, des polysorbates, des octyl- ou nonylphénols, des esters glycériques du macrogol ou des cétomacrogols ou des substances du groupe des Poloxamers® ou leurs mélanges.

8. Procédé de préparation de microparticules selon la revendication 2, **caractérisé en ce qu'**on utilise comme émulsifiant (s) dispersible (s) dans l'eau de la phosphatidycholine ou du palmitate-stéarate 15 de sucrose ou leurs mélanges.

9. Procédé de préparation de microparticules selon la revendication 2, **caractérisé en ce qu'**on utilise comme solvant inoffensif du point de vue de la santé pour les polyesters un alcool inférieur, de préférence l'alcool éthylique.

10. Procédé de préparation de microparticules selon la revendication 2, **caractérisé en ce qu'**on utilise comme liquide contenant du gaz de l'eau ou du glycérol à 87 %.

11. Procédé de préparation de microparticules selon la revendication 1 ou respectivement 2 , **caractérisé en ce qu'**on utilise comme milieu de suspension de l'eau pour injection, éventuellement avec addition de chlorure de sodium et/ou de glucose et/ou de mannitol et/ou de lactose, qui contient éventuellement en outre une substance tensioactive selon la revendication 13 et/ou un polyalcool.

12. Agents de contraste pour examens par ultrasons, **caractérisés en ce que** les microparticules selon la revendication 3 ou respectivement 4 sont remises en suspension dans un milieu de suspension pharmaceutiquement acceptable.

13. Agents de contraste pour examens par ultrasons selon la revendication 12, **caractérisés en ce qu'**on utilise comme milieu de suspension pharmaceutiquement acceptable de l'eau pour injection qui contient éventuellement une addition de chlorure de sodium, de glucose, de mannitol et/ou de lactose et éventuellement en outre une substance tensioactive selon la revendication 7. et/ou du polyalcool.

14. Agents de contraste pour examens par ultrasons qui peuvent être préparés selon le procédé décrit dans les revendications 1 et 2, **caractérisés en ce qu'**on omet une lyophilisation finale.
